# EUROPEAN PATENT APPLICATION

(11) **EP 2 436 300 A1**
(43) Date of publication of application: **04.04.2012**
(21) Application number: 11180448.0
(22) Date of filing: 07.09.2011
(51) Int. Cl.: A61B 1/005, A61B 1/018, A61B 1/04, A61B 1/273, A61B 1/00, A61B 1/06

(54) **Endoscope apparatus**

(30) Priority: 30.09.2010 JP 2010223293
(71) Applicant: Fujifilm Corporation, Minato-ku Tokyo (JP)
(72) Inventor: IWASAKA, Masayuki, Kanagawa (JP); OHKI, Tomohiro, Kanagawa (JP)
(74) Representative: Klunker . Schmitt-Nilson . Hirsch

(57) **Abstract**

An endoscope apparatus (100) includes an endoscope insertion portion (13), an operation portion (11) provided at the side of the base end of the endoscope insertion portion, an operating wire (57) for transmitting an operation of the operation portion (11) with a tensile force, and a bending portion (19) provided at a distal-end side of the endoscope insertion portion to be subjected to a bending operation performed with the operating wire (57). The bending portion (19) is such that, when a bending-portion cross-section defined by a plane perpendicular to a longitudinal axis of the endoscope insertion portion (13) is divided into two regions (63,65) by a straight line (61) passing through the center (59) of the bend-portion cross-section, the bend-portion cross-section is dividable into a first region (63) in which a cross-section of the operating wire (57) is present and a second region (65) in which the cross-section of the operating wire (57) is not present. A distal-end projection domain is obtained by projecting the first region (63) in which the cross-section of the operating wire (57) is present onto a distal-end surface (37) at a distal-end of the endoscope insertion portion (13). The distal-end projection domain is protruded from the distal-end of the endoscope insertion portion outward in a direction of the longitudinal axis of the endoscope insertion portion (13). An observation window (39) for observing an object is arranged in the protruded distal-end projection domain.

## Description

### BACKGROUND OF THE INVENTION

### 1. Technical Field

The present invention relates to an endoscope apparatus.

### 2. Description of Related Art

An endoscope apparatus is known, which is configured to insert a distal-end part of an endoscope insertion portion into a narrow body cavity tube (see JP-A-2004-351138). This endoscope apparatus is such that the distal-end part is protruded like a spatula, that the tapered spatula-like distal-end part is inserted into the body cavity tube while pressed against a living-body tissue, and that thus, the insertability thereof into a stenosed body cavity tube is enhanced. Another endoscope apparatus is known, which is configured so that the diameter of a distal-end hard part is reduced to enhance the insertability thereof into the narrow body cavity tube (see JP-A-2009-213603). This endoscope apparatus is maintained and fixed in a position in which a light guide is raised at a predetermined angle from the direction of an axis line thereof by a guiding cap from a fixing cap attached to the distal-end hard part. Thus, the light guide is prevented from interfering with other members. In addition, the diameter of the distal-end hard part is reduced. Another endoscope is known, which is configured so that manipulations performed with a treatment instrument can easily be observed (see JP-A-8-071030). This endoscope apparatus is configured such that a treatment instrument protruding port is arranged at the side of the inner circumference of the bend of an insertion portion with respect to an observation window. Thus, treatment instruments are protruded to the center of an observation view field. Consequently, the treatment instruments can easily reach a target diseased site.

### SUMMARY

However, the endoscope apparatus whose distal-end part is shaped like a spatula is still large in insertion resistance, due to the spatula-shaped distal-end part, to be inserted into a particularly narrow body cavity tube such as a bile duct or a pancreatic duct. Even if the diameter of the distal-end hard part is reduced by preventing the light guide from interfering with another member, the distal-end hard part may be expanded depending upon circumstances. Thus, the diameter of the distal-end hard part can be more reduced. In addition, in a configuration in which the treatment instrument is protruded to the center of the observation view field, the direction of the protruded treatment instrument cannot be fine adjusted. Accordingly, sometimes, it is difficult to observe manipulations performed with treatment instruments.

An object of the invention is to provide an endoscope apparatus configured such that a distal-end of an endoscope insertion portion can smoothly be fit into small-diameter bile and pancreatic ducts, that the visibility of an observation view field can be enhanced, and that manipulations performed with treatment instruments can easily be observed.

The invention is constituted by the following elements.

An endoscope apparatus includes an endoscope insertion portion, an operation portion, an operating wire and a bend portion. The endoscope insertion portion has a distal-end and a base end and a longitudinal axis. The operation portion is provided at a side of the base end of the endoscope insertion portion. The operating wire transmits an operation of the operation portion with a tensile force. The bend portion is provided at the distal-end side of the endoscope insertion portion to be subjected to a bending operation performed with the operating wire. The bend portion is such that, when a bend-portion cross-section defined by a plane perpendicular to the longitudinal axis of the endoscope insertion portion is divided into two regions by a straight line passing through a center of the bend-portion cross-section, the bend-portion cross-section is dividable into a first region in which a cross-section of the operating wire is present and a second region in which the cross-section of the operating wire is not present. A distal-end projection domain is obtained by projecting the first region in which the cross-section of the operating wire is present onto a distal-end surface at the distal-end of the endoscope insertion portion. The distal-end projection domain is protruded from the distal-end of the endoscope insertion portion outward in a direction of the longitudinal axis of the endoscope insertion portion. An observation window which observes an object is arranged in the protruded distal-end projection domain.

In accordance with the endoscope apparatus according to the invention, a distal-end of an endoscope insertion portion can smoothly be fit into small-diameter bile and pancreatic ducts. The visibility of an observation view field can be enhanced. Manipulations performed with treatment instruments can easily be observed.

### BRIEF DESCRIPTION OF THE DRAWINGS

Fig. 1 is a view for illustrating an embodiment of the invention, more particularly, an external view of an endoscope apparatus according to the invention.
FIG. 2 is a perspective view of an endoscope distal-end portion of the endoscope apparatus illustrated in FIG. 1.
FIG. 3 is a front view of a distal-end surface in plan view of the endoscope distal-end portion of the endoscope apparatus illustrated in FIG. 2.
FIG. 4 is an enlarged cross-sectional view of a bending portion of the endoscope apparatus illustrated in FIG. 1.
FIG. 5 illustrates a front view of a vicinity of a duodenum, and a partially cutaway enlarged view of a primary part of the vicinity of the duodenum.
FIG. 6A is a side view of the endoscope distal-end portion that faces a duodenal papilla when a body cavity is cut. FIG. 6B is a side view of the endoscope distal-end portion inserted into a bile duct.
FIG. 7 is a cross-sectional view of the endoscope distal-end portion having a forceps port on the periphery of which a raising support is provided.
FIG. 8A is a cross-sectional view of a distal-end hard portion provided with the raising support that is tilted to change the direction of protruding a treatment instrument into a view field of an observation window. FIG. 8B is a cross-sectional view of the distal-end hard portion provided with the raising support set to extend horizontally to change the direction of protruding the treatment instrument into an ordinary direction along an axis line of the endoscope distal-end portion.
FIG. 9 is a cross-sectional view of a distal-end hard portion formed by bending a treatment instrument insertion channel.
FIG. 10A is a cross-sectional view of a distal-end hard portion provided with a raising support extending along a treatment instrument insertion channel to change the direction of protruding a treatment instrument into a view field of an observation window. FIG. 10B is a cross-sectional view of the distal-end hard portion provided with the raising support set to extend horizontally to change the direction of protruding the treatment instrument into an ordinary direction along an axis line of the endoscope distal-end portion.

### DETAILED DESCRIPTION OF EMBODIMENTS OF THE INVENTION

Hereinafter, an embodiment of the invention is described with reference to the drawings.

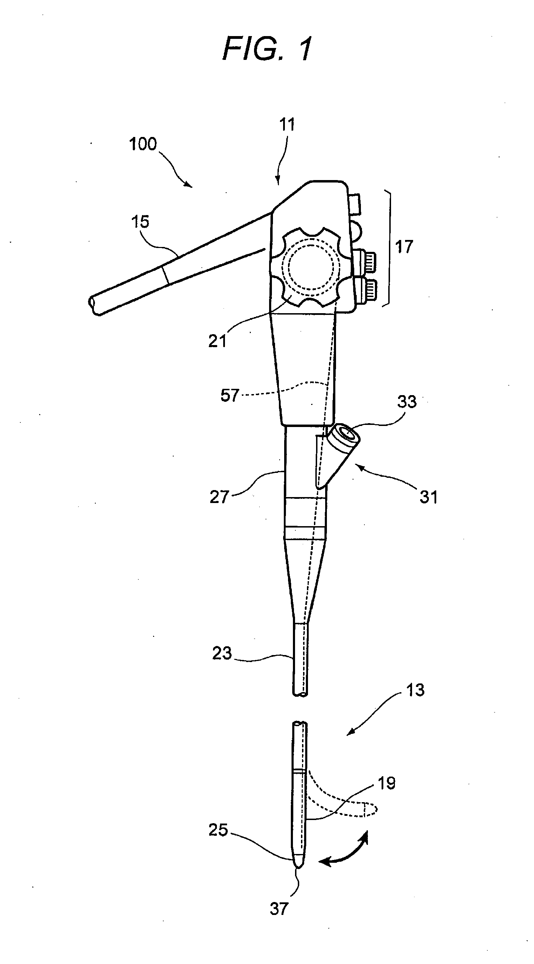

Fig. 1 is a view for illustrating an embodiment of the invention, and, more particularly, an external view of an endoscope apparatus according to the invention.

An endoscope apparatus 100 includes an endoscope operation portion 11, and an endoscope insertion portion 13 that has a base end portion provided continuously to the endoscope operation portion 11 and that is inserted into a body cavity from a leading end portion serving as a distal-end portion thereof. A universal cord 15 containing various duct lines and signal cables is connected to the endoscope operation portion 11. A connector to be detachably connected to a control apparatus (not shown) is attached to a distal-end portion of the universal cord 15. Various buttons 17, such as a suction button, a shutter button, and a function switching button, are provided on the endoscope operation portion 11 to be juxtaposed with one another. In addition, an angle knob 21 for bending a bending portion 19 provided at the distal-end side of the endoscope insertion portion 13.

The endoscope insertion portion 13 is provided with a soft portion 23, a bending portion 19, and a distal-end portion (endoscope distal-end portion) 25, which are arranged in a longitudinal direction from the side of the endoscope operation portion 11 in this order. The soft portion 23 has flexibility and is provided continuously to a base end side of the bending portion 19. The bending portion 19 is bent by turning the angle knob 21 of the endoscope operation portion 11 to pull a bending-portion operating wire inserted into the endoscope insertion portion 13. Thus, an operation of bending the bending portion 19 in a single direction can be performed by a tensile force of the operating wire. Accordingly, the endoscope distal-end portion 23 can be directed to a desired direction.

A continuously provided portion 27 between the endoscope operation portion 11 and the endoscope insertion portion 13 is provided with a treatment instrument insertion portion 33 for inserting a treatment instrument into a treatment instrument insertion channel 31 into which a treatment instrument, a water-supply means and the like are inserted. The treatment instrument insertion channel 31 is formed to extend to the endoscope distal-end portion 25 from the endoscope operation portion 11 such that various treatment instruments such as a high-frequency scalpel can be inserted thereinto.

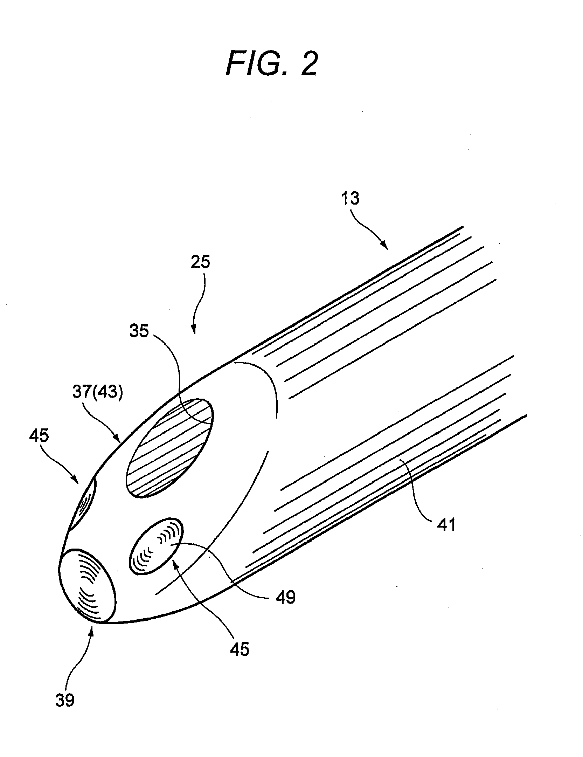

FIG. 2 is a perspective view of the endoscope distal-end portion 25 illustrated in FIG. 1.

An observation window 39 for observing a test-object, and a forceps port 35 into which a treatment instrument 29 is inserted are arranged on a distal-end surface 37 of the endoscope insertion portion 13 of the endoscope apparatus 100. The observation window 39 is arranged at an axial distal-end of the endoscope insertion portion 13 to protrude therefrom. The distal-end surface 37 extends from the observation window 39 to a part at which the distal-end surface 37 is connected to an outer circumferential surface 41 of the endoscope insertion portion 13, and is formed of a tapered slope 43 from which the observation window 39 is protruded. The entire circumferential distal-end surface from the observation window 39 to the outer circumferential surface 41 of the endoscope insertion portion 13 is configured by a tapered slope 43. Consequently, the distal-end of the endoscope insertion portion 13 is sharpened. Thus, the insertion of the distal end of the endoscope insertion portion 13 into the particularly narrow body cavity is enabled, so that the range of utilization of the endoscope apparatus 100 can be expanded.

The forceps port 35 is opened in the slope 43. Accordingly, the forceps port 35 can be arranged utilizing the slope 43 configured to provide good insertability. Thus, the insertability is not reduced by the forceps port 35. In addition, a pair of illumination windows 45 is not arranged on the slope 43. Even in this case, the insertability is not lowered by the illumination windows 45.

Each illumination window 45 is arranged halfway between the observation window 39 and the forceps port 35 on the slope 43. The pair of illumination windows 45 can be arranged utilizing marginal spaces respectively formed at both sides of the boundary portion at which the circular observation window 39 and the circular forceps port 35 are located close to each other. Accordingly, efficient arrangement of the illumination windows 45 can be implemented, which prevents occurrence of wasted spaces.

Each illumination window 45 has an outer surface inclined along the distal-end surface 37. A cover lens 49 is provided at the distal end portion of an illumination through hole. The outer surface of the cover lens 49 is flush with the slope 43. With such a configuration, each illumination window 45 becomes part of the slope 43. Thus, the endoscope distal-end portion 25 can smoothly be inserted into the duct.

The endoscope apparatus 100 outputs illumination light from the pair of illumination windows 45 and acquires an observation image through the observation window 39. The light output from a light source portion of the control apparatus is supplied to the endoscope apparatus 100 through the connector and the universal cord 15. The supplied light is irradiated from the illumination windows 45 of the endoscope distal-end portion 25 through the light guide.

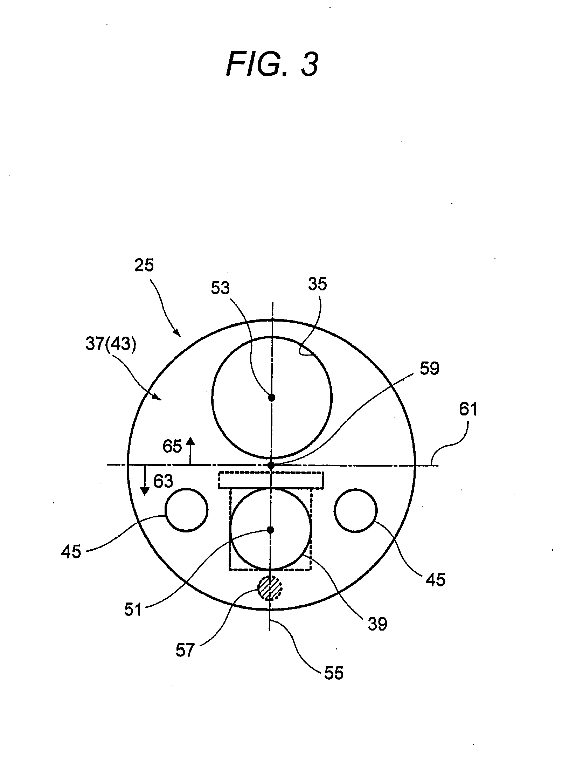

FIG. 3 is a front view illustrating the distal-end surface 37 in plan view of the endoscope distal-end portion 25 illustrated in FIG. 2.

According to the present example of the configuration, when a bending-portion cross-section of the bending portion 19, which is defined by a plane perpendicular to a longitudinal axis of the endoscope insertion portion 13, is divided into two regions by a straight line 61 passing through the center 59 of the bending-portion cross-section, the bending-portion cross-section is dividable into a first region 63 in which a cross-section of the operating wire 57 is present, and a second region 65 in which no cross-section of the operating wire 57 is present.

Then, a distal-end projection domain is obtained by projecting, onto a distal-end surface 37 at the distal-end of the endoscope insertion portion 13, the first region 63 in which a cross-section of the operating wire 57 is present. The distal-end projection domain, i.e., the first region 63 of the distal-end surface 37 appearing in front view in FIG. 3 is protruded from the distal-end of the endoscope insertion portion 13 outward in a direction of a longitudinal axis of the endoscope insertion portion 13.

The configuration of the endoscope distal-end portion 25 is more specifically described hereinafter. In the endoscope distal-end portion 25 in front view, the pair of illumination windows 45 is arranged on a straight line intersecting with a straight line 55 passing through the center of the observation window 39 and that of the forceps port 35. The operating wire 57 is arranged at the side opposite to the forceps port 35 across the observation window 39. That is, the operating wire 57 is arranged only in the above first region 63 at the side of the observation window 39. On the other hand, the forceps port 35 is arranged in the second region 65.

That is, the straight line 61 intersects with the straight line 55 connecting the observation window center 51 and the forceps port center 53 to each other in the endoscope distal-end portion 25 in axial front view. In addition, the straight line 61 is a line that divides into two regions the endoscope distal-end surface of the endoscope distal-end portion 25 in axial front view. The observation window 39 and the operating wire 57 are arranged in one of the two regions, i.e., the first region 63 using the straight line 61 as the boundary between the two regions. On the other hand, the forceps port 35 is arranged in the other region, i.e., the second region 65.

With such an arrangement, a waste of installation space of each of the observation window 39 and the illumination windows 45 is eliminated. Then, the saved space can be appropriated for the forceps port 35. Consequently, a large space can be assured as the installation space of the forceps port 35.

The bending portion 19 is bent by being pulled with the above operating wire 57 such that a side of the bending portion 19, in which the operating wire 57 is arranged, is set to be an inner side of the bend of the bending portion 19. Accordingly, the operating wire 57 is arranged opposite to the forceps port 35 across the observation window 39. The operating wire 57, the observation window 39, and the forceps port 35 are sequentially arranged from the inner side in a bending direction toward an outer side of the bend of the bending portion 19. On the other hand, the endoscope distal-end portion 25 is such that a part thereof at the inner side of the bend is protruded.

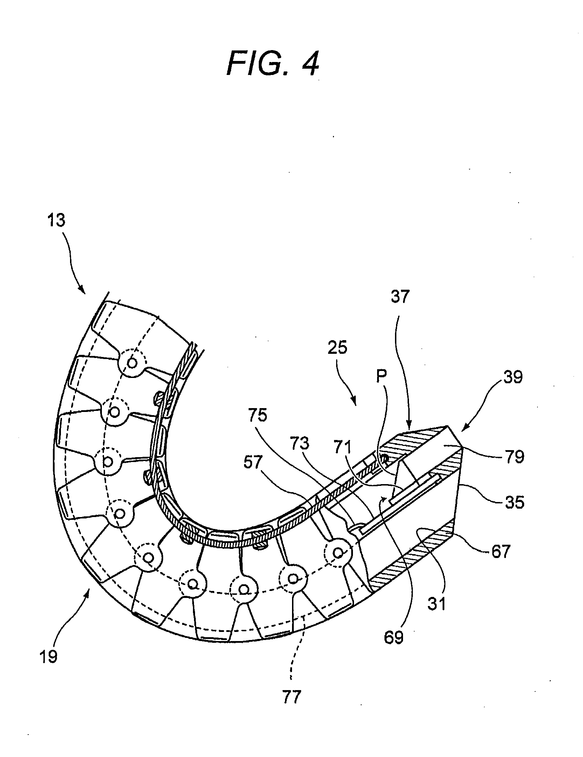

FIG. 4 is an enlarged cross-sectional view illustrating the bend portion 19 of the endoscope apparatus 100 illustrated in FIG. 1.

The endoscope distal-end portion 25 has a circular-cylindrically-shaped distal-end hard portion 67 made of, e.g., ceramics, and stainless steel. An imaging optical system 69 is provided in the distal-end hard portion 67. The imaging optical system 69 includes an imaging device 71 for imaging an observed region illuminated with an illumination optical system, and a substrate 73 on which the imaging device 71 is mounted. An imaging signal representing an observed image obtained from the imaging device 71 is output to the control apparatus through a signal line 75 connected to the substrate 73. The control apparatus causes a display portion (not shown) to display image information obtained by performing image processing on the imaging signal input thereto. Instructions representing such a sequence of processing can be input from an input portion, such as a keyboard or a mouse, connected to the control apparatus. A charge-coupled device (CCD) image sensor or a complementary metal-oxide semiconductor (CMOS) image sensor is used as the imaging device 71 of the imaging optical system 69.

The imaging optical system 69 provided inside the observation window 39 is such that an image is formed on the imaging device 71 mounted on the substrate 73 from light taken in from an objective lens group configured by a plurality of objective lenses housed in a lens unit 79 by turning the optical path of the light a right angle with a prism P. Image signals based on the image information taken in by the imaging device 71 is output through the above signal line 75 connected to the substrate 73. With this configuration, the imaging device 71 can be arranged in parallel with the central axis of the endoscope distal-end portion 25, using the prism P. Thus, the imaging device 71 and the lens unit 79 can be housed efficiently in space and compactly. Consequently, the diameter of the endoscope distal-end portion 25 is reduced.

The treatment instrument insertion channel 31 is passed through the distal-end hard portion 67. The treatment instrument insertion channel 31 is opened in the distal-end surface 37 as the forceps port 35. In the illustrated example, the treatment instrument insertion channel 31 is formed to be parallel to the axis line of the distal-end hard portion 67. Thus, a treatment instrument passed through the treatment instrument insertion channel 31 and led out of the forceps port 35 is protruded in parallel to the axis line of the distal-end hard portion 67.

The operating wire 57 is caused by a pulling operation performed with the angle knob 21 of the endoscope operation portion 11 (see FIG. 1) to bend the bending portion 19. A resin tube 77 configuring the treatment instrument insertion channel 31 is arranged along the longitudinal direction of the endoscope insertion portion 13 on the outer side of the bend of the bending portion 19. The resin tube 77 is connected to the distal-end hard portion 67 at a distal-end side thereof and communicates with the treatment instrument insertion portion 33 at a base-end side thereof. When the operating wire 57 is loosened, the resin tube 77 and the members such as various signal lines housed in the endoscope insertion portion 13 cancel the bent-state of the bending portion 19 due to an elastically restoring force thereof.

Next, endoscopy of a bile duct is described hereinafter as an example of an operation of the endoscope apparatus 100 having the above configuration.

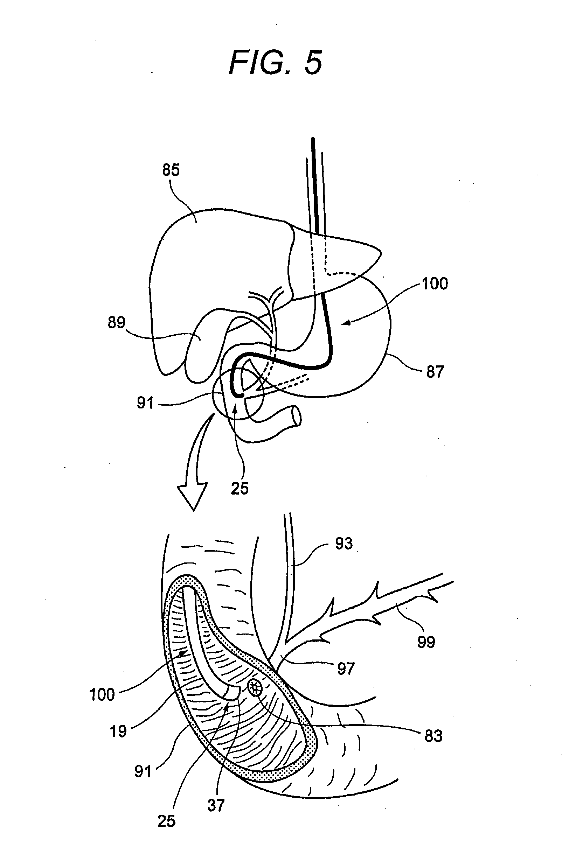

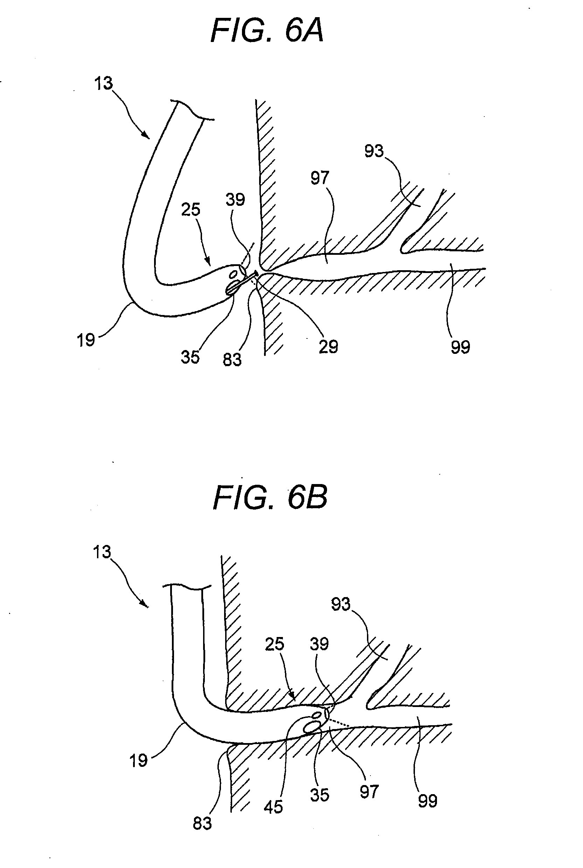

FIG. 5 illustrates a front view of a liver 85, a stomach 87, and a cholecyst 89 in the vicinity of a duodenum 91, and a partially cutaway enlarged view of a primary part of each of the liver 85, the stomach 87, and the cholecyst 89 in the vicinity of the duodenum 91. FIG. 6A is a side view of the endoscope distal-end portion 25 that faces a duodenal papilla 83 when a body cavity is cut. FIG. 6B is a side view of the endoscope distal-end portion 25 inserted into a bile duct 93.

The distal-end portion 25 of the endoscope apparatus 100 is inserted to the position of the duodenum 91 by an operator. A common duct 97 to which the bile duct 93 and the pancreatic duct 99 are connected is connected to the duodenum 91. At the connection region between the common duct 97 and the duodenum 91, a duodenal papilla 83 is opened. The operator operates the angle knob 21 to inflect the bending portion 19. As illustrated in FIG. 6A, the endoscope distal-end portion 25 is arranged to face the duodenal papilla 83.

Then, the operator causes the treatment instrument, which is a high-frequency scalpel, to protrude from the forceps port 35. While viewing an imaged image obtained through the observation window 39, the operator cuts the duodenal papilla 83 with the high-frequency scalpel. Upon completion of cutting the duodenal papilla 83, the operator inserts the endoscope distal-end portion 25 into the cut duodenal papilla 83, as illustrated in FIG. 6B. Then, the operator inserts the endoscope distal-end portion 25 into the duodenal papilla 83, and into the bile duct 93 from the common duct 97. Thus, the endoscopic observation of the inside of the bile duct 93 is performed.

With this configuration, when cutting the duodenal papilla 83 located at the inner side of the bend, the view field can be assured without hiding the boundary between a cut part and an uncut part with the treatment instrument 29. In the bent-state illustrated in FIG. 4, the duodenal papilla 83 is located in front of or below the observation window 39. In a case where cutting proceeds towards an upper part illustrated in FIG. 4, if the positions of the observation window 39 and the forceps port 35 are exchanged, the boundary between the cut part and the uncut part is hidden by the treatment instrument 29. According to this configuration, the manipulation performed with the treatment instrument 29 can surely be observed, without occurrence of such a situation.

Thus, the endoscope can smoothly be inserted into the particularly narrow body cavity tube by sharpening the distal end of the endoscope insertion portion 13. In addition, the distal-end projection domain (i.e., the first region 63 of the distal-end surface 37) located at the inner side of the bend of the bending portion is protruded from the distal-end of the endoscope insertion portion 13 outward in a direction of a longitudinal axis of the endoscope insertion portion 13. Consequently, as compared with a case where the outer side of the bend thereof is protruded, the extent of the bend of the bending portion 19 is small. Accordingly, advancing/retreating operations of the treatment instrument to be inserted into the treatment instrument insertion channel 31 can smoothly be performed. The operability of the treatment instrument can be enhanced.

The endoscope distal-end portion 25 is not protruded more frontwardly than the observation window 39. Thus, the observation view field is not obstructed by a protruded part, so that the visibility of the view field can be enhanced. For example, when the endoscope distal-end portion 25 is inserted into the narrow body cavity tube, observation can be performed at the most distal end of the inserted distal-end portion 25. Thus, the inside of the body cavity tube into which the endoscope distal-end portion 25 is inserted can quickly be observed. In addition, even when not the entire endoscope distal-end portion 25 is inserted, the cavity tube into which the endoscope distal-end portion 25 is inserted can be observed. Thus, the inside of each of the various narrow body cavity tubes can quickly be observed. Consequently, the usability of the endoscope apparatus can be enhanced.

In addition, the observation window 39 is arranged at the most distal-end of the endoscope distal-end portion 25. Thus, the assurance of the arrangement space of the imaging optical system 69 can be facilitated. Consequently, the distal-end hard portion 67 can be shortened.

Next, other examples of the configuration of the endoscope distal-end portion are described hereinafter.

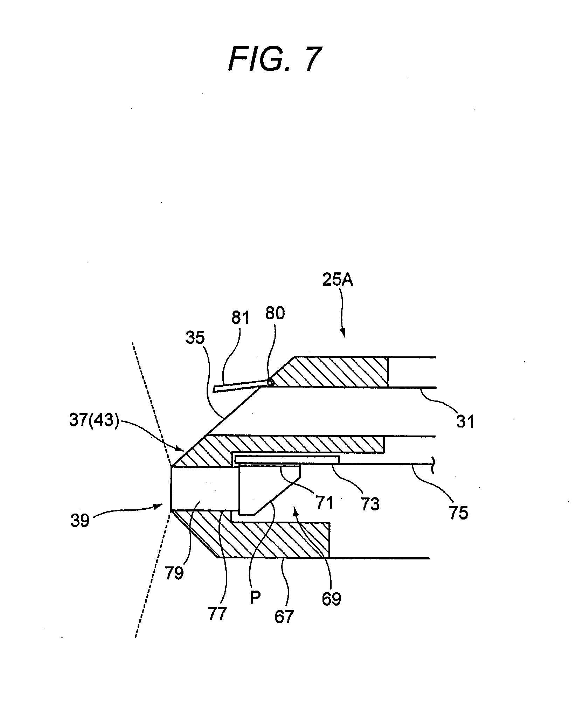

FIG. 7 is a cross-sectional view taken on a straight line 55 illustrated in FIG. 3.

A raising support 81 for controlling a direction of protruding a treatment instrument protruded from the forceps port 35 is arranged on the periphery of the forceps port 35. The raising support 81 is tilted around a hinge portion 80 by a raising support wire (not shown). The raising support 81 is supported tiltably from a central axis of the forceps port 35 to the side of the observation window 39. Accordingly, the direction of protruding the treatment instrument 29 to be protruded from the forceps port 35 can be fine adjusted according to the inclination of the raising support 81.

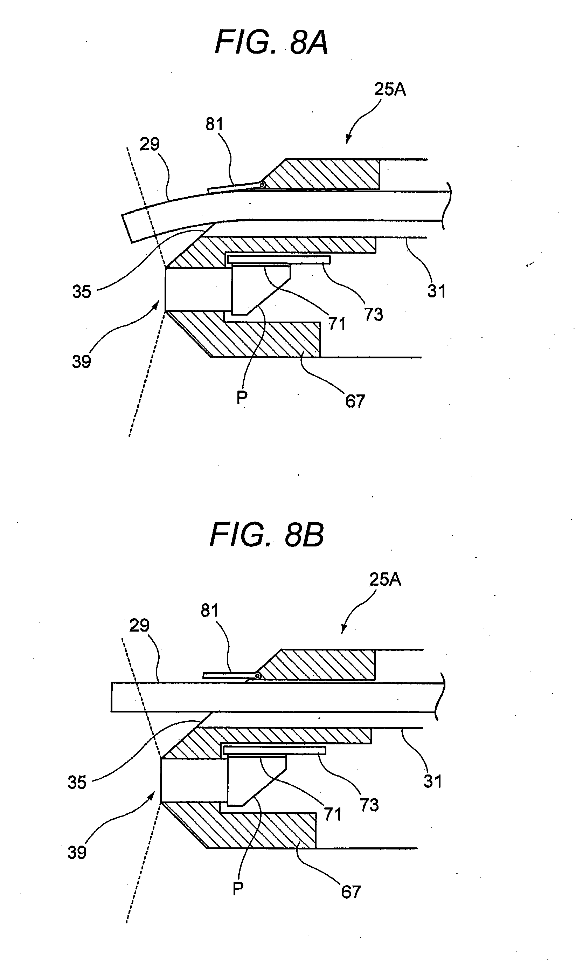

FIG. 8A is a cross-sectional view illustrating the distal-end hard portion 67 provided with the raising support 81 that is tilted to change the direction of protruding the treatment instrument 29 into the view field of the observation window 39. FIG. 8B is a cross-sectional view illustrating the distal-end hard portion 67 provided with the raising support 81 set to extend horizontally to change the direction of protruding the treatment instrument 29 into an ordinary direction along an axis line of the endoscope distal-end portion 25.

According to this example, the raising support 81 has the hinge portion 80 arranged opposite to the observation window 39. The treatment instrument 20 protruded from the forceps port 35 can selectively be tilted to the side of the observation window 39 by tilting the raising support 81 around the hinge portion 80. The direction of protruding the treatment instrument 29 can be changed to the view field center of the observation window 39 by tilting the raising support 81, as illustrated in FIG. 8A.

Generally, an operation for arranging the distal end of the treatment instrument 29 at a target part in a body cavity is performed by performing operations of advancing and retreating the treatment instrument 29 or an operation of bending the distal-end bending portion of the endoscope insertion portion 13 at an operator-side of the endoscope apparatus 100. However, in a body cavity whose inside diameter is substantially no different from the outside diameter of the endoscope insertion portion 13, the distal-end bending portion cannot be largely bent. In addition, in a case where a lesion to be extracted is minute, and where the selection of the position of the distal end of the treatment instrument 29 and the determination of the direction thereof need delicate adjustment operations, sometimes, it is difficult to implement such operations by the bending with the bending portion 19. According to the present embodiment, the raising support is provided in the apparatus. The direction of protruding the treatment instrument 29 can be fine adjusted. The operations are facilitated. In addition, more complicated treatments can be achieved.

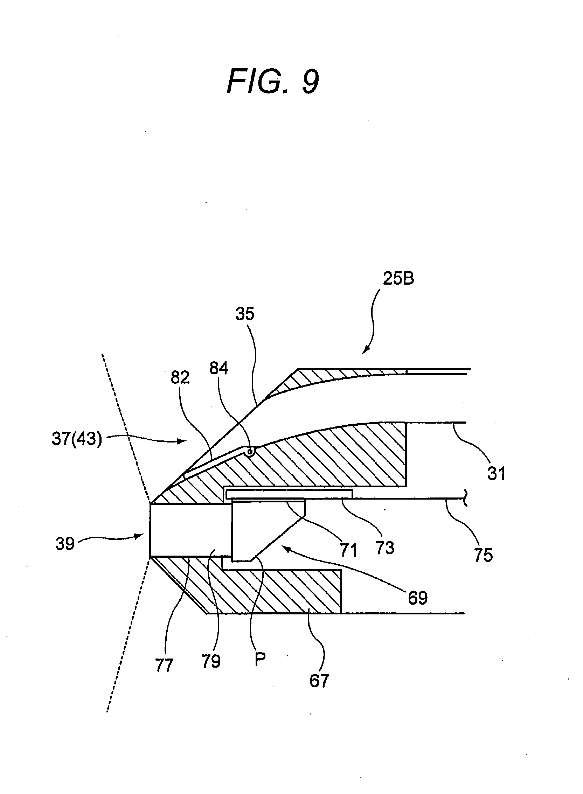

FIG. 9 is a cross-sectional view illustrating the distal-end hard portion 67 formed by bending the treatment instrument insertion channel 31.

With this configuration of the endoscope distal-end portion 25, vicinity of the forceps port 35 of the treatment instrument insertion channel 31 in which the forceps port 35 is opened is formed by bending the channel 31 so that the channel 31 diminishes in distance between the channel 31 and the observation window 39 towards the forceps port 35. With this configuration, the direction of protruding the treatment instrument 29, which is passed through the treatment instrument insertion channel 31 and protruded from the forceps port 35, is restricted by the direction of bending the treatment instrument insertion channel 31 and changed to a direction directed to the center of the view field of the observation window 39.

With the above configuration, the raising support 82 supported by the hinge portion 84 is arranged at the side of the observation window 39 of the forceps port 35. The raising support 82 is supported tiltably from the central axis of the forceps port 35 towards a side opposite to the side of the observation window 39. Consequently, the treatment instrument 29 protruded from the forceps port 35 can selectively be moved apart from the side of the observation window 39.

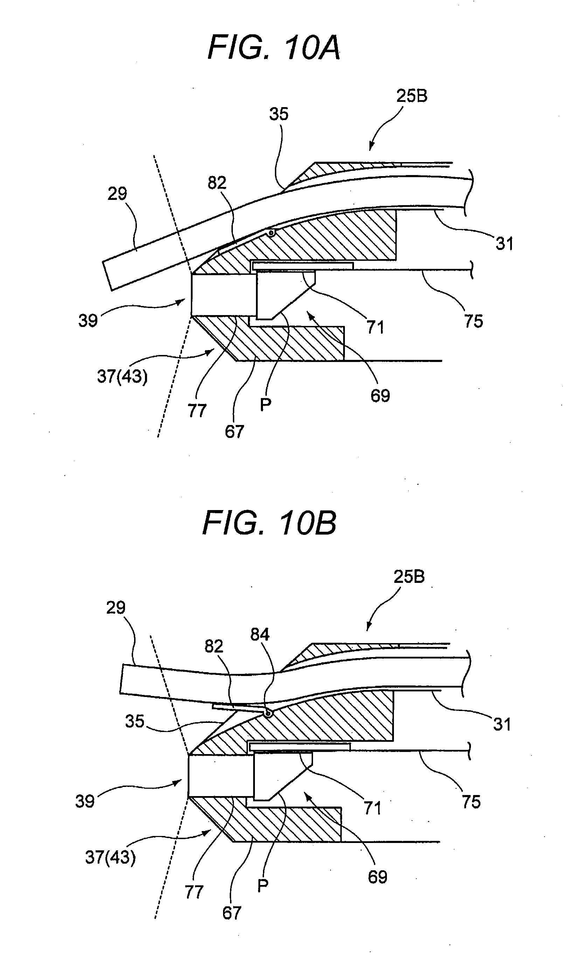

FIG. 10A is a cross-sectional view illustrating the distal-end hard portion 67 provided with the raising support 82 extending along a treatment instrument insertion channel 31 to change, into a view field of the observation window 39, the direction of protruding the treatment instrument 29. FIG. 10B is a cross-sectional view illustrating the distal-end hard portion 67 provided with the raising support 82 set to extend horizontally to change, into an ordinary direction along an axis line of the endoscope distal-end portion 25, the direction of protruding the treatment instrument 29.

With this configuration, the treatment instrument 29 passes through the treatment instrument insertion channel 31. Thus, the direction of protruding the treatment instrument 29 is changed to a direction towards the view field center of the observation window 39. That is, the raising support 82 arranged at the side of the observation window 39 is tilted around the hinge portion 84. Consequently, as illustrated in FIG. 10B, the direction of protruding the treatment instrument 29 can be changed to a direction in which the treatment instrument 29 is moved apart from the side of the observation window 39, that is, the ordinary direction of a straight line along an axis line of the endoscope distal-end portion 25.

Accordingly, according to the configuration of the above endoscope distal-end portion 25, the manipulations performed with the treatment instrument 29 are facilitated while the visibility of the observation view field is enhanced.

Thus, the invention is not limited to the above embodiments. Modifications and applications made by those skilled in the art, based on the description of the specification and the known techniques may occur in the invention, and are included in the scope of claims for patent.

As described above, the following items are disclosed in the present specification.
(1) An endoscope apparatus includes an endoscope insertion portion, an operation portion, an operating wire and a bend portion. The endoscope insertion portion has a distal-end and a base end and a longitudinal axis. The operation portion is provided at a side of the base end of the endoscope insertion portion. The operating wire transmits an operation of the operation portion with a tensile force. The bend portion is provided at the distal-end side of the endoscope insertion portion to be subjected to a bending operation performed with the operating wire. The bend portion is such that, when a bend-portion cross-section defined by a plane perpendicular to the longitudinal axis of the endoscope insertion portion is divided into two regions by a straight line passing through a center of the bend-portion cross-section, the bend-portion cross-section is dividable into a first region in which a cross-section of the operating wire is present and a second region in which the cross-section of the operating wire is not present. A distal-end projection domain is obtained by projecting the first region in which the cross-section of the operating wire is present onto a distal-end surface at the distal-end of the endoscope insertion portion. The distal-end projection domain is protruded from the distal-end of the endoscope insertion portion outward in a direction of the longitudinal axis of the endoscope insertion portion. An observation window which observes an object is arranged in the protruded distal-end projection domain.

According to this endoscope apparatus, the first region in which a cross-section of the operating wire is present is projected onto the distal-end projection domain. Then, the distal-end projection domain is protruded from the distal-end of the endoscope insertion portion outward in a direction of a longitudinal axis of the endoscope insertion portion. Thus, the insertion of the distal end of the endoscope insertion portion into the particularly narrow body cavity tube can smoothly be performed. The range of utilization of the endoscope apparatus can be expanded. In addition, the observation window is arranged on the protruded distal-end projection domain. Thus, the observation view field can more surely be assured without obstructing the observation view field.
(2) The endoscope apparatus according to (1), the distal-end surface between an edged of the observation window and an edge of an outer circumferential surface of the endoscope insertion portion is a sloping surface to form an end of the endoscope apparatus having a taper shape so as to protrude the observation window.

According to this endoscope apparatus, a part extending from the observation window to the outer circumferential surface of the endoscope insertion portion is formed of a tapered slope. Consequently, the distal end of the endoscope insertion portion is sharpened. Thus, the insertion of the distal end of the endoscope insertion portion into the particularly narrow body cavity tube can be performed.
(3) The endoscope apparatus according to (2), a forceps port is opened in the sloping surface.

According to this endoscope apparatus, the forceps port can be arranged utilizing the slope that provides good insertability thereof. Thus, the insertability is not lowered by the forceps port.
(4) The endoscope apparatus according to any one of (1) to (3), an imaging optical system is arranged inside the observation window. The imaging optical system has a light collecting lens, an imaging device, and a prism which is arranged to face a light receiving surface of the imaging device and to guide imaging-light traveling from the light collecting lens to the light receiving surface by bending an optical axis at a right angle.

According to this endoscope apparatus, the imaging device to be arranged inside the observation window, and the light collecting lens can be housed efficiently in space and compactly.
(5) The endoscope apparatus according to any one of (1) to (4), the sloping surface has an illumination window from which illumination light is irradiated onto the object.

According to the endoscope apparatus, the illumination window can be arranged utilizing the slope that provides good insertability. The insertability is not lowered by the illumination window.
(6) The endoscope apparatus according to (5), the illumination window is arranged on a slope halfway from the observation window to the forceps port.

According to this endoscope apparatus, a pair of illumination windows can be arranged utilizing marginal spaces respectively formed at both sides of the boundary portion at which the circular observation window and the circular forceps port are located close to each other. Accordingly, efficient arrangement of the illumination windows can be implemented, which prevents occurrence of wasted spaces on a limited distal-end space.
(7) The endoscope apparatus according to any one of (3) to (6), a raising support is provided on periphery of the forceps port to control a direction in which a treatment instrument to be protruded from the forceps port is protruded.

According to this endoscope apparatus, the direction of protruding the treatment instrument from the forceps port can be fine adjusted by tilting the raising support.
(8) The endoscope apparatus according to (7), the raising support has a hinge portion arranged on a side opposite to the observation window across the forceps port. The raising support is supported by the hinge portion so as to incline the treatment instrument from a central axis of the forceps port towards a side of the observation window.

According to this endoscope apparatus, the direction of protruding the treatment instrument can be changed to a direction towards the view field center of the observation window by tilting the raising support to the side of the observation window.
(9) The endoscope apparatus according to (8), a treatment instrument insertion channel leads to the forceps port. A part of the treatment instrument insertion channel decreases in distance between the part of the treatment instrument insertion channel and the observation window towards the forceps port.

According to this endoscope apparatus, the direction of protruding a treatment instrument passed through the treatment instrument insertion channel and protruded from the forceps port is restricted by the direction of bending the treatment instrument insertion channel and changed to a direction directed to the center of the view field of the observation window.
(10) The endoscope apparatus according to any one of (7) to (9), the raising support has another hinge portion arranged on a side of the observation window around the forceps port. The raising support is supported by said hinge portion so as to incline the treatment instrument from the central axis of the forceps port towards a side opposite to the observation window.

According to this endoscope apparatus, a treatment instrument protruded from the forceps port can selectively be moved apart from the side of the observation window. That is, the direction of protruding the treatment instrument can be changed, from a direction in which the treatment instrument insertion channel extends, to the ordinary direction of a straight line extending along an axis line of the endoscope distal-end portion.

## Claims

1. An endoscope apparatus comprising:
an endoscope insertion portion that has a distal-end, a base end and a longitudinal axis;
an operation portion that is provided at a side of the base end of the endoscope insertion portion;
an operating wire that transmits an operation of the operation portion with a tensile force; and
a bend portion that is provided at the distal-end side of the endoscope insertion portion to be subjected to a bending operation performed with the operating wire,
wherein the bend portion is such that, when a bend-portion cross-section defined by a plane perpendicular to the longitudinal axis of the endoscope insertion portion is divided into two regions by a straight line passing through a center of the bend-portion cross-section, the bend-portion cross-section is dividable into a first region in which a cross-section of the operating wire is present and a second region in which the cross-section of the operating wire is not present,
wherein a distal-end projection domain is obtained by projecting the first region in which the cross-section of the operating wire is present onto a distal-end surface at the distal-end of the endoscope insertion portion,
wherein the distal-end projection domain is protruded from the distal-end of the endoscope insertion portion outward in a direction of the longitudinal axis of the endoscope insertion portion, and
wherein an observation window which observes an object is arranged in the protruded distal-end projection domain.

2. The endoscope apparatus according to claim 1,
wherein the distal-end surface between an edged of the observation window and an edge of an outer circumferential surface of the endoscope insertion portion is a sloping surface to form an end of the endoscope apparatus having a taper shape so as to protrude the observation window.

3. The endoscope apparatus according to claim 2,
wherein a forceps port is opened in the sloping surface.

4. The endoscope apparatus according to any one of claims 1 to 3,
wherein an imaging optical system is arranged inside the observation window, and
wherein the imaging optical system has a light collecting lens, an imaging device, and a prism which is arranged to face a light receiving surface of the imaging device and to guide imaging-light traveling from the light collecting lens to the light receiving surface by bending an optical axis at a right angle.

5. The endoscope apparatus according to any one of claims 1 to 4,
wherein the sloping surface has an illumination window from which illumination light is irradiated onto the object.

6. The endoscope apparatus according to claim 5,
wherein the illumination window is arranged on a slope halfway from the observation window to the forceps port.

7. The endoscope apparatus according to any one of claims 3 to 6,
wherein a raising support is provided on periphery of the forceps port to control a direction in which a treatment instrument to be protruded from the forceps port is protruded.

8. The endoscope apparatus according to claim 7,
wherein the raising support has a hinge portion arranged on a side opposite to the observation window across the forceps port, and
wherein the raising support is supported by the hinge portion so as to incline the treatment instrument from a central axis of the forceps port towards a side of the observation window.

9. The endoscope apparatus according to claim 8,
wherein a treatment instrument insertion channel leads to the forceps port, and wherein a part of the treatment instrument insertion channel decreases in distance between the part of the treatment instrument insertion channel and the observation window towards the forceps port.

10. The endoscope apparatus according to claim 9,
wherein the raising support has another hinge portion arranged on a side of the observation window around the forceps port, and
wherein the raising support is supported by said hinge portion so as to incline the treatment instrument from the central axis of the forceps port towards a side opposite to the observation window.
